# EUROPÄISCHE PATENTANMELDUNG

(11) **EP 0 908 195 A2**
(43) Veröffentlichungstag der Anmeldung: **14.04.1999**
(21) Anmeldenummer: 98890286.2
(22) Anmeldetag: 07.10.1998
(51) Int. Cl.: A61M 35/00

(54) **Einrichtung zum lokalen Auftragen flüssiger oder pastöser Medien auf die Körperhaut**

(30) Priorität: 08.10.1997 AT 1707/97
(71) Anmelder: Haslauer, Paul, A-5020 Salzburg (AT)
(72) Erfinder: Haslauer, Paul, A-5020 Salzburg (AT)
(74) Vertreter: Köhler-Pavlik, Johann, Dipl.-Ing.

(57) **Zusammenfassung**

Die Erfindung betrifft eine Einrichtung zum lokalen Anlegen flüssiger oder pastöser Medien auf die Körperhaut, insbesondere auf die Kopfhaut, umfassend eine über die entsprechende Hautstelle anlegbare Folie (1). Um zu gewährleisten, daß ein zur Pflege oder zu therapeutischen Zwecken anzuwendendes Medium rasch, einfach und sicher auf die gewünschte Hautstelle ohne bzw. geringe Beeinflussung der Umgebung aufgebracht werden kann, und daß ferner das Medium gut dosierbar und auf der aufgebrachten Stelle verteilbar ist, ist vorgesehen, daß die Folie Stellen (2) zum Einbringen der Medien zwischen Körperhaut und Folie (1) aufweist. Die Folie (1) ist vorteilhafterweise aus elastischem Material und beispielsweise zur Behandlung der Kopfhaut in Form einer Haube, allenfalls mit Krempe (12) ausgebildet. Die Stellen (2) zum Einbringen des Mediums unter die Folie (1) weisen vorteilhafterweise geringere Dicke auf und sind zur Verstärkung durch einen Wulst (3) od. dgl. umgeben. Allenfalls können vorgefertigte Löcher (13) an den Stellen (2) vorgesehen sein.

## Beschreibung

Die Erfindung betrifft eine Einrichtung zum lokalen Auftragen flüssiger oder pastöser Medien auf die Körperhaut, insbesondere auf die Kopfhaut, umfassend eine über die entsprechende Hautstelle anlegbare Folie.

Es gibt eine Reihe von kosmetischen oder therapeutischen Anwendungen, bei welchen flüssige oder pastöse Medien äußerlich auf die Körperhaut aufgebracht werden. Beispielsweise werden auf die Haut Salben oder Flüssigkeiten aufgetragen, um die Haut direkt zu beeinflussen oder über die Haut in den Körper einzudringen und dort bestimmte Wirkungen zu entfalten. Es ist auch bekannt, flüssige Medien zur Stärkung der Haarwurzeln zu verwenden.

Dabei tritt das Problem auf, daß bei manuellem Auftragen der gewünschten Medien die Finger der Person, welche das Medium aufträgt mit diesem benetzt werden. Durch die Verwendung von Handschuhen oder Instrumenten zum Auftragen kann dieser Nachteil wohl vermieden werden, jedoch um den Preis, daß die Handhabung oder Behandlung, insbesondere beim Einmassieren des Mediums, beeinträchtigt wird.

Als weiterer Nachteil wäre zu nennen, daß ein lokal begrenztes Auftragen insbesondere flüssiger oder pastöser Medien mit niedriger Viskosität nicht oder nur beschränkt möglich ist, da das Medium über die Grenzen des gewünschten Gebiets "fließt" und an nicht gewünschten Lokalisationen unerwünschte Wirkungen hervorrufen kann. Insbesondere soll bei der Anwendung an der Kopfhaut ein Kontakt des Mediums mit den Augen verhindert werden. Zur Verstärkung der Wirkung des Mediums auf die Haut bzw. die darunterliegenden Regionen ist oft auch ein Einmassieren vorteilhaft. Durch das Einmassieren werden die Grenzen des gewünschten Hautareals ebenfalls überschritten.

Darüberhinaus kann als weiterer unerwünschter Effekt bei der Anwendung flüssiger oder pastöser Medien eine Kältewirkung aufgrund der Verdunstung des Flüssigkeitsgehalts in dem Medium auftreten. Dies führt auch dazu, daß das Medium zu rasch austrocknet.

Bei längeren Anwendungen ist es für die betreffende Person sehr unangenehm in einer bestimmten Stellung auszuharren.

In der heutigen Zeit ist ein besorgniserregender Anstieg verschiedener Allergien und Hautkrankheiten zu verzeichnen, die häufigere kosmetische oder therapeutische Behandlungen notwendig machen. Neben einer Reihe von Hautunverträglichkeitsreaktionen seien als Beispiele Psoriasis (Schuppenflechte) und Neurodermitis genannt. Zur Behandlung von Psoriasis werden stark photosensibilisierende Lösungen angewendet, weshalb sich die betroffenen Personen durch Handschuhe und Sonnenbrillen vor Sonnenbestrahlung besonders schützen müssen.

Die FR-PS 2 693 657 beschreibt eine geschmeidige Maske, durch welche flüssiges oder gasförmiges Medium zwischen Gesicht und Maske eingebracht werden kann. Bei dieser Einrichtung hat die Maske eine bestimmte, dem Gesicht der zu behandelnden Person annähernd entsprechende Form und hat nicht die Anpassungsfähigkeit einer elastischen Folie.

Die Aufgabe der vorliegenden Erfindung besteht daher in der Schaffung einer Einrichtung, durch welche gewährleistet wird, daß ein zur Pflege oder zu therapeutischen Zwecken od. dgl. anzuwendendes Medium an eine Körperhautstelle rasch, einfach und sicher ohne bzw. mit nur geringer Beeinflussung der Umgebung der zu behandelnden Hautstelle aufgebracht werden kann. Mit der Einrichtung soll weiters das Medium gut dosierbar und auf der aufgebrachten Stelle verteilbar, z.B. einmassierbar sein. Außerdem soll auch die Umgebung der zu behandelnden Hautstellen im wesentlichen unbeeinflußt bleiben. Die oben erwähnten Nachteile sollen vermieden oder zumindest reduziert werden.

Gelöst wird die Aufgabe dadurch, daß eine elastische, die zu behandelnde Körperstelle vorzugsweise randseitig dicht umschließende Folie vorgesehen ist. Dadurch wird weitgehend verhindert, daß die zu kosmetischen oder therapeutischen Zwecken verwendeten Medien über die gewünschte Stelle der Haut austreten. Die flüssigen oder pastösen Medien können auf der körperhautseitigen Fläche der Folie vor dem Aufbringen derselben auf die zu behandelnde Körperstelle aufgetragen und unter besonders günstigen Übergangsbedingungen auf die Körperhaut, insbesondere Kopfhaut, appliziert werden und beispielsweise durch Einmassieren eine bessere Wirkung erzielt werden.

Nach einem weiteren Merkmal der Erfindung weist die Folie Stellen, z.B zum Einstechen einer Nadel od.dgl., durch welche das Behandlungsmittel eingebracht wird, Einstiche, Löcher od.dgl. zum Einbringen der Medien zwischen Körperhaut und Folie mit einer solchen Nadel, auf. Durch diese Maßnahme ist es möglich, daß man beim Einmassieren mit dem Medium nicht in Kontakt kommt - abgesehen von geringfügigen Mengen aufgrund von Undichtheiten z.B. der Löcher zum Einbringen des Mediums. Insbesondere bei der behaarten Kopfhaut wird durch das Einmassieren der durch das Haar bewirkte Strohdacheffekt überwunden, sodaß das Medium an der Kopfhaut seine volle Wirkung entfalten kann. Die Einrichtung eignet sich für eine Reihe von kosmetischen oder therapeutischen Medien, welche über eine bestimmte Zeit auf einen bestimmten Bereich der Haut aufgebracht werden sollen.

Um eine gute Anpassung der Folie an die jeweilige Körperregion zu gewährleisten, ist die Folie aus gummieleastischem Material, z.B. Silikongummi, gebildet. Dadurch wird auch sichergestellt, daß die lokal begrenzte Region der Haut an der das Medium wirken soll, abgedichtet ist. Die Verwendung von Silikon hat noch dazu den Vorteil der Sterilisierbarkeit aufgrund der hohen Hitzebeständigkeit.

Wenn an jener, der Körperhaut zugewandten Seite der Folie eine Zwischenfolie aus anderem Material als die Folie angeordnet ist, können Hautunverträglichkeitsreaktionen gegen das Material der Folie vermieden werden. So können beispielsweise Allergien gegen Silikon durch Zwischenlage einer sehr dünnen Polyäthylenfolie vermieden werden. Darüberhinaus wird die Folie durch die Zwischenfolie, welche als Wegwerfartikel ausgebildet sein kann, geschont.

Für die Anwendung an der Kopfhaut ist die Folie vorteilhafterweise in Form einer Haube, vorzugsweise mit einer Krempe od. dgl. ausgebildet. Die Krempe hat den Vorteil, daß etwaiges außerhalb der haubenförmigen Folie austretendes Medium sich darin sammelt und beispielsweise nicht in das Gesicht der zu behandelnden Person gerät. Darüberhinaus erleichtert die Krempe das Aufsetzen der Haube. Vorteilhafterweise ist die Krempe mit der haubenförmigen Folie einstückig hergestellt.

Ebenso kann die Folie für die Anwendung beispielsweise an Extremitäten in Form eines Schlauches ausgebildet sein, der über die Extremität gezogen wird.

Gemäß einem weiteren Merkmal der Erfindung kann die Folie auch durch elastische Bänder od. dgl. an die entsprechende Hautstelle anpreßbar gestaltet sein. Beispielsweise kann durch die Anwendung hochelastischer Gummibänder eine haubenförmig gestaltete Folie auch an Köpfe von Kindern angepreßt werden oder auch andere Regionen der Körperhaut entsprechend abgedeckt werden.

Zur Vermeidung von Rissen durch das Einstechen von Nadeln an den Stellen zum Einbringen der Medien, können dort vorgefertigte Löcher vorgesehen sein. Diese sollten so groß sein, daß ein Einstecken der Nadel zum Einbringen des flüssigen oder pastösen Mediums möglich ist, aber gleichzeitig möglichst klein, daß durch eine Ventilwirkung möglichst wenig Medium austreten kann.

Um ein gleichmäßiges Einbringen des Mediums unter die Folie zu gewährleisten, sind die Stellen zum Einbringen der Medien vorteilhafterweise äquidistant verteilt.

Wenn die Folie an den Stellen zum Einbringen der Medien geringere Dicke aufweist als im übrigen Bereich, wird das Einstechen beispielsweise einer Einstechspitze erleichtert.

Zur Verhinderung des Einreißens der Folie an den Stellen zum Einbringen der Medien sind diese gemäß einem weiteren Erfindungsmerkmal von einer Struktur mit größerer Dicke als die übrige Folie, beispielsweise einem Wulst od. dgl. umgeben.

Durch Strukturen an jener, der Haut zugewandten Seite der Folie können bestimmte Massageeffekte oder gezielte Verteilungen des Mediums an der jeweiligen Körperstelle erzielt werden. Der Gestalt und Anordnung der Strukturen sind dabei keine Grenzen gesetzt.

Um ein Auslaufen der Medien über das gewünschte Hautareal zu verhindern, ist an jener, der Haut zugewandten Seite der Folie randseitig ein Dichtungsstreifen od. dgl. vorgesehen. Um eine billige Herstellung der Einrichtung zu erzielen sind die Strukturen und ein allfälliger Dichtungsstreifen vorteilhafterweise in einem Stück mit der Folie hergestellt.

Weitere Einzelheiten der Erfindung werden anhand der beigefügten Zeichnungen näher erläutert.

Darin zeigen
- Fig. 1: eine schematische Darstellung der Anwendung der Erfindung in Form einer Haube zur Behandlung der Kopfhaut mit einer Einrichtung zur Zuführung des Behandlungsmediums,
- Fig. 2: die Draufsicht auf einen Ausschnitt der Haube aus Fig. 1 mit einer Stelle zum Einbringen des Medium in größerem Maßstab, und
- Fig. 3: einen Schnitt durch den Ausschnitt der Haube gemäß Fig. 2 entlang der Schnittlinie III-III.

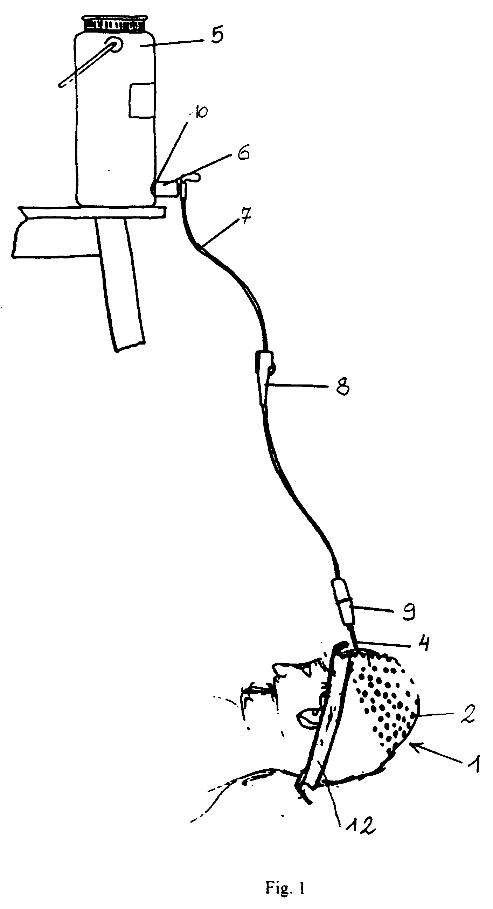

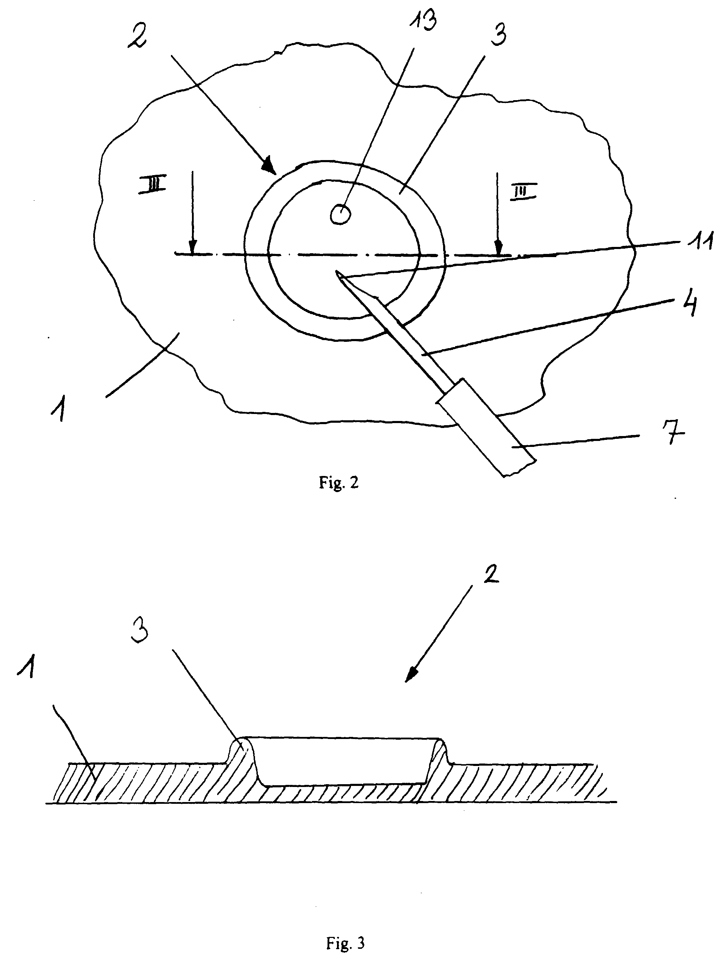

Fig. 1 zeigt die erfindungsgemäße Einrichtung zum lokalen Anlegen flüssiger oder pastöser Medien auf die Kopfhaut in Form einer zu einer Haube geformten Folie 1, welche aus Silikongummi oder anderen elastischen Materialien hergestellt sein kann. Neben der hervorragenden Elastizität von Silikon kann dieses Material wegen seiner Hitzebeständigkeit auch sterilisiert werden. Durch die Elastizität des Materials der haubenförmigen Folie 1 ist diese an den Kopf anpreßbar. Theoretisch könnte die Folie 1 auch aus nicht elastischem Material bestehen und das Anpressen an die Kopfhaut durch andere Mittel, wie z.B. aufblasbaren Bereichen an der Folie 1 in der Art eines Schwimmflügels geschehen. Zum leichteren Aufsetzen der haubenförmigen Folie 1 ist der Rand derselben mit einer Krempe 12 versehen, welcher randseitig in einem dickeren Wulst endet, der die Haube vor Einreißen schützt. Zusätzlich kann auch ein Dichtungsstreifen (nicht dargestellt) vorgesehen sein, damit ein dichter Sitz der Haube am Kopf gewährleistet wird. Ein solcher Dichtungsstreifen ist vorteilhafterweise durch eine entsprechende Struktur, wie z.B. einen Wulst an der, der Haut zugewandten Seite der Haube gebildet. Die haubenförmige Folie 1 weist eine Vielzahl Stellen 2 zur Einbringung des flüssigen oder pastösen Mediums zwischen die Kopfhaut und die Folie 1 auf, welche vorteilhafterweise äquidistant verteilt sind. Die Stellen 2 zum Einbringen der Medien können verschiedenartig ausgebildet sein. Eine bevorzugte Ausführungsform wird weiter unten anhand der Abbildungen 2 und 3 beschrieben. Das zum Auftragen auf die Kopfhaut bestimmte Medium wird in einem Behälter 5 aufbewahrt. Zur Anwendung wird dieser Behälter 5 umgelegt, sodaß sich die Auslaßöffnung 10 an der höchsten Stelle befindet. Nach Entfernen eines allfälligen Verschlusses für Transportzwecke (nicht dargestellt), wird ein Auslaufhahn 6 in die Auslaßöffnung 10 eingesetzt. Über den Auslaufhahn 6 wird ein Schlauch 7 geschoben, durch den das Behandlungsmedium über einen Dosierregler 8 und eine Förderpumpe 9 einer Kanüle 4 zugeführt wird. Die Kanüle 4 wird an den Stellen 2 zum Einbringen des Mediums beispielsweise in die Folie eingestochen und eine gewünschte Menge des Mediums in die haubenförmig angeordnete Folie eingespritzt. Um einen geeigneten Fluß des Mediums in dem Schlauch 7 zu gewährleisten, muß der Behälter in einer ausreichenden Höhe über dem Kopf der zu behandelnden Person, in ähnlicher Weise wie bei einer Infusion positioniert werden. Durch den Dosierregler 8 kann die Menge des eingebrachten Mediums geregelt werden. Je nach Erfordernis wird das Medium nur an einer Stelle 2 oder an mehreren Stellen 2 unter die Folie 1 eingebracht.

Fig. 2 zeigt einen Ausschnitt der Folie 1 in der Umgebung einer Stelle 2 zum Einbringen der Medien zwischen Körperhaut und Folie 1 in größerem Maßstab. Gemäß dieser bevorzugten Ausführungsform ist die Stelle 2 dadurch gekennzeichnet, daß sie geringere Dicke als die übrige Folie 1 aufweist, wie in Fig. 3 ersichtlich. In diesem Ausführungsbeispiel wird das Medium über einen Schlauch 7 und eine Kanüle 4, welche mit einer Einstechspitze 11 versehen ist, unter die Folie 1 eingebracht. Zu diesem Zweck durchsticht die Einstechspitze 11 der Kanüle 4 die Folie 1 an der Stelle 2 mit geringerer Dicke der Folie 1. Nach dem Herausziehen der Kanüle 4 schließt sich das Loch durch die Elastizität der Folie selbständig, wie es beispielsweise in Form einer Membran bei Impfstoffen, welche von der Injektionsnadel durchstochen wird der Fall ist. Ebenso kann ein kleines vorgefertigtes Loch 13 an der Stelle 2 zum Einbringen des Mediums vorgesehen sein, durch das die Einstechspitze 11 gezwungen wird. Das Loch 13 verringert die Wahrscheinlichkeit des Einreißens der Folie 1. Als weiterer Schutz vor Einreißen der Folie 1 im Bereich der Stelle 2 mit reduzierter Dicke kann randseitig, eine Struktur mit größerer Dicke, z.B. in Form eines ringförmig um die Stelle 2 angeordneten Wulstes 3 vorgesehen sein. Zusätzlich bildet der Wulst 3 eine Art Führung für die Einstechspitze 11, welche das gezielte Einstechen erleichtert.

Durch die erfindungsgemäße Einrichtung kann das Medium sicher unter die Folie auf die entsprechende Körperhautstelle z.B. Kopfhaut aufgebracht werden. Das Medium kann in die Haut einmassiert werden ohne daß die Finger mit dem Medium in Kontakt kommen. Dadurch kann verhindert werden, daß die behandelnde Person das Medium durch Unachtsamkeit in die Augen reibt und mit den Schleimhäuten in Berührung kommt. Durch den Abschluß des Mediums kühlt und trocknet dieses weniger rasch aus. Weiters ist durch das ventilartige Verschließen der Löcher 3 gewährleistet, das allfällige Dämpfe des Behandlungsmediums nicht austreten und Schleimhäute oder Augen irritieren könnten.

Zum besseren oder gezielten Verteilen bzw. Einmassieren des Mediums in die gewünschten Hautstellen können auch an jener, der Haut zugewandten Seite der Folie 1 entsprechende Strukturen vorgesehen sein. Um eine einfachere Herstellung zu gewährleisten sind diese Strukturen vorteilhafterweise mit der Folie in einem Stück produziert.

Die Ausführung der erfindungsgemäßen Einrichtung als Haube ist als bevorzugtes Ausführungsbeispiel gedacht und kann beispielsweise zur Behandlung der Kopfhaut mit einem gegen Psoriasis wirkenden Mittel verwendet werden. Es liegt aber im Rahmen der Erfindung, beliebige andere Ausführungsformen der Einrichtungen zu wählen. So ist es beispielsweise möglich, mit gleichem Effekt die Einrichtung als Handschuh oder als Schlauch auszubilden, welcher über bestimmte Stellen der Arme, Beine oder Körperteile gezogen wird, wobei die offenen Ränder allenfalls mit einem Dichtungsstreifen versehen sein können, ähnlich wie bei der Kopfhaube erwähnt wurde. Schließlich besteht auch die Möglichkeit, die Einrichtung als Streifen, Band od. dgl. auszuführen und mit einem Verschluß, z.B. Klettverschluß zu versehen und damit die Möglichkeit zu bieten, die zu behandelnde Stelle mit dem Band zu umschließen und durch den Klettverschluß einen Schlauch zu bilden. Auch in diesem Fall kann insbesondere, wenn das Band den zu behandelnden Körperteil nicht rutschfest umschließt, randseitig ein Dichtungsstreifen vorgesehen sein, durch welchen einerseits ein Halt des Bandes am betreffenden Körperteil gesichert und anderseits ein ungewolltes Austreten des Mediums oder von

## Patentansprüche

1. Einrichtung zum lokalen Anlegen flüssiger oder pastöser Medien auf die Körperhaut, insbesondere auf die Kopfhaut, umfassend eine über die entsprechende Hautstelle anlegbare Folie, dadurch gekennzeichnet, daß eine elastische, die zu behandelnde Körperstelle vorzugsweise randseitig dicht umschließende Folie (1) vorgesehen ist.

2. Einrichtung nach Anspruch 1, dadurch gekennzeichnet, daß die Folie (1) Stellen, z.B. zum Einstechen einer Nadel od.dgl., durch welche das Behandlungsmittel eingebracht wird, Einstiche, Löcher od.dgl. zum Einbringen der Medien zwischen Körperhaut und Folie mit einer solchen Nadel, aufweist.

3. Einrichtung nach Anspruch 1 oder 2, dadurch gekennzeichnet, daß die Folie (1) aus elastischem, vorzugsweise gummielastischem Material, z.B. Silikongummi, gebildet ist.

4. Einrichtung nach mindestens einem der Ansprüche 1 bis 3, dadurch gekennzeichnet, daß an jener, der Körperhaut zugewandten Seite der Folie (1) eine Zwischenfolie aus anderem Material als die Folie (1) angeordnet ist.

5. Einrichtung nach mindestens einem der Ansprüche 1 bis 4, dadurch gekennzeichnet, daß die Folie (1) in Form einer Haube, vorzugsweise mit einer Krempe (12) od. dgl. ausgebildet ist.

6. Einrichtung nach mindestens einem der Ansprüche 1 bis 4, dadurch gekennzeichnet, daß die Folie (1) in Form eines Schlauches ausgebildet ist.

7. Einrichtung nach mindestens einem der Ansprüche 1 bis 6, dadurch gekennzeichnet, daß die Folie (1) durch elastische Bänder od. dgl. an die entsprechende Hautstelle anpreßbar ist.

8. Einrichtung nach mindestens einem der Ansprüche 1 bis 7, dadurch gekennzeichnet, daß die Stellen (2) zum Einbringen der Medien äquidistant verteilt sind.

9. Einrichtung nach mindestens einem der Ansprüche 1 bis 8, dadurch gekennzeichnet, daß die Folie (1) an den Stellen (2) zum Einbringen der Medien geringere Dicke aufweist als im übrigen Bereich.

10. Einrichtung nach mindestens einem der Ansprüche 1 bis 9, dadurch gekennzeichnet, daß die Stellen (2) zum Einbringen der Medien von einer Struktur mit größerer Dicke als die übrige Folie (1), beispielsweise einem Wulst (3) od.dgl. umgeben ist.

11. Einrichtung nach mindestens einem der Ansprüche 1 bis 10, dadurch gekennzeichnet, daß an jener, der Haut zugewandten Seite der Folie (1) Strukturen vorgesehen sind.

12. Einrichtung nach mindestens einem der Ansprüche 1 bis 11, dadurch gekennzeichnet, daß an jener, der Haut zugewandten Seite der Folie (1) randseitig ein Dichtungsstreifen od.dgl. vorzugsweise einstückig mit der Folie (1) vorgesehen ist.
